(19) Europäisches Patentamt
European Patent Office
Office européen des brevets

(11) EP 3 112 390 A1

(12) **EUROPEAN PATENT APPLICATION**
published in accordance with Art. 153(4) EPC

(43) Date of publication:
**04.01.2017 Bulletin 2017/01**

(21) Application number: **15754427.1**

(22) Date of filing: **23.02.2015**

(51) Int Cl.:
***C08G 64/04*** (2006.01)
***C07C 39/16*** (2006.01)
***C08K 5/524*** (2006.01)
***C08L 69/00*** (2006.01)
***C08L 83/04*** (2006.01)

(86) International application number:
**PCT/JP2015/055090**

(87) International publication number:
**WO 2015/129638 (03.09.2015 Gazette 2015/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.02.2014 JP 2014039502**

(71) Applicant: **Idemitsu Kosan Co., Ltd**
**Tokyo 100-8321 (JP)**

(72) Inventor: **WATANABE, Nobuhiro**
**Ichihara-shi**
**Chiba 299-0193 (JP)**

(74) Representative: **Hoffmann Eitle**
**Patent- und Rechtsanwälte PartmbB**
**Arabellastraße 30**
**81925 München (DE)**

(54) **POLYCARBONATE RESIN, AND POLYCARBONATE RESIN COMPOSITION**

(57) Provided is a polycarbonate resin, including, as a dihydric phenol serving as a raw material, a bisphenol A having an isopropenylphenol concentration of 150 ppm by mass or less within 1 hour after granulation and under an ambient temperature condition of from 10°C to 50°C.



Printed by Jouve, 75001 PARIS (FR)

## Description

TECHNICAL FIELD

The present invention relates to a polycarbonate having a satisfactory hue, and more specifically, to a polycarbonate resin having a reduced yellow tint (low YI value) and an excellent light transmission property.

BACKGROUND ART

A polycarbonate resin (hereinafter sometimes abbreviated as "PC resin") is excellent in, for example, transparency, mechanical characteristics, thermal stability, electrical properties, and weatherability, and hence has been used in an optical molded article, such as a light-guiding plate, a lens, or an optical disc, by taking advantage of such characteristics. However, the light transmission property of the resin is lower than that of a polymethyl methacrylate (PMMA) or the like, and hence its color tone has a slightly yellow tint.

Particularly in the case where the light-guiding length of a light-guiding plate using the resin becomes longer, when the material has a yellow tint, a light beam having a shorter wavelength is absorbed. Accordingly, there occurs a phenomenon in which a difference in color tone between a portion close to a light source and a portion distant therefrom is observed, and there occurs a problem in that the color tone becomes nonuniform. A polycarbonate material having a reduced yellow tint has been required for solving such problem.

With regard to a light-guiding plate or the like, the thinning of such product has been progressing. Accordingly, there has been a growing requirement for a molding material having high flowability and suppressed in yellowing in injection molding at high temperature.

A thin-walled product obtained by injection molding at high temperature has heretofore been typically applied to, for example, a digital versatile disc (DVD). In the application, however, required quality in terms of a color tone is not as high as that in a light-guiding member because a required light transmission length is as short as the thickness direction of the disc (about 0.6 mm).

In Patent Document 1, there is a disclosure of a method involving setting the total amount of specific eight kinds of impurities in a polycarbonate resin to 750 ppm by mass or less to reduce the number of white spots in an optical disc substrate and an error rate therein.

In Patent Document 2, there is a disclosure of a polycarbonate resin composition for an optical disc substrate containing a polycarbonate resin produced by using, as a raw material, 2,2-(4-hydroxyphenyl)propane (bisphenol A) in which the content of a cyclic dimer of *p*-isopropenylphenol is 150 ppm by mass or less and the content of trisphenol is 150 ppm by mass or less, and 100 ppm to 500 ppm of a release agent, the resin composition having a feature in that the viscosity-average molecular weight of the resin composition in a pelletized state is from 10,000 to 17,000, and the hydroxyl group terminal content thereof is less than 7 mol%. The amount of 2-(2-hydroxyphenyl)-2-(4-hydroxyphenyl)propane (2,4-isomer) in the polycarbonate resin composition is 1,000 ppm or less.

In Patent Document 3, there is a disclosure of a method of producing an aromatic PC including the step of retaining the molten state of the composition of bisphenol A and phenol, which is obtained in an apparatus for producing bisphenol A, under high temperature with an inert gas (nitrogen gas) without granulating the composition, followed by the supply of the composition to a subsequent PC production apparatus. In Patent Document 3, there is a disclosure that the content of 4-isopropenylphenol in the composition of 2, 2- (4-hydroxyphenyl)propane and phenol retained in a molten state to be subjected to the polymerization of the polycarbonate is 1,000 ppm or less.

CITATION LIST

PATENT DOCUMENT

[Patent Document 1] JP 4621312 B2
[Patent Document 2] JP 2001-344813 A
[Patent Document 3] JP 2002-173530 A

SUMMARY OF THE INVENTION

TECHNICAL PROBLEM

As disclosed in Patent Documents 1 to 3, various attempts have been made to obtain a polycarbonate resin

having a reduced amount of impurities through, for example, the improvement of a production method for a polycarbonate resin. However, it is difficult to achieve a low level of YI value applicable to a light-guiding member even by those methods. The present invention has been made to solve such problems, and an object of the present invention is to provide a polycarbonate resin that has a satisfactory hue and achieves such a YI value that the resin can be suitably used as a light-guiding member, and a composition thereof.

SOLUTION TO PROBLEM

Isopropenylphenol is a substance incorporated as an impurity into bisphenol A serving as a raw material together with, for example, the 2,4-isomer of bisphenol A. Isopropenylphenol has been known as a decomposition product of bisphenol A, and has been known as a substance having high reactivity. It has been known that when bisphenol A is left to stand in air, bisphenol A changes into other impurities, such as a cyclic dimer in which two isopropenylphenol molecules are bonded to each other, and trisphenol produced by a reaction between bisphenol A and isopropenylphenol. The inventors of the present invention have found that even when the isopropenylphenol concentration itself of bisphenol A serving as a raw material before the production of a polycarbonate is low, concern is raised in that an impurity produced by the change of isopropenylphenol in bisphenol A serving as a raw material is responsible for an influence on the hue of the polycarbonate resin.

In view of the foregoing, the inventors have paid attention to the fact that the isopropenylphenol concentration becomes lower with time, and have found that a polycarbonate resin having a satisfactory hue can be produced by using, as a raw material, a bisphenol A having an isopropenylphenol (hereinafter sometimes abbreviated as "IPP") concentration equal to or less than a specific value within 1 hour after granulation and under an ambient temperature condition of from 10°C to 50°C.

That is, the present invention includes the following.

1. A polycarbonate resin, comprising, as a dihydric phenol serving as a raw material, a bisphenol A having an isopropenylphenol concentration of 150 ppm by mass or less within 1 hour after granulation and under an ambient temperature condition of from 10°C to 50°C.
2. The polycarbonate resin according to Item 1, wherein the polycarbonate resin has a viscosity-average molecular weight of from 9,000 to 17,500.
3. A polycarbonate resin composition, comprising 100 parts by mass of (A) a polycarbonate resin containing 60 mass% or more of the polycarbonate resin of Item 1 or 2, and 100 ppm by mass to 1,500 ppm by mass of (B) a phosphorus-based antioxidant.
4. The polycarbonate resin composition according to Item 3, wherein (B) the phosphorus-based antioxidant has a pentaerythritol structure.
5. The polycarbonate resin composition according to Item 3 or 4, wherein (B) the phosphorus-based antioxidant comprises bis(2,6-di-*tert*-butyl-4-methylphenyl)pentaerythritol diphosphite and/or bis(2,4-dicumylphenyl)pentaerythritol diphosphite.
6. The polycarbonate resin composition according to any one of Items 3 to 5, further comprising, with respect to 100 parts by mass of (A) the polycarbonate resin, 200 ppm by mass to 1,500 ppm by mass of (C) a polyorganosiloxane having a functional group.
7. The polycarbonate resin composition according to Item 6, wherein the functional group comprises at least one selected from the group consisting of an alkoxy group, an aryloxy group, a polyoxyalkylene group, a carboxyl group, a silanol group, an amino group, a mercapto group, an epoxy group, and a vinyl group.
8. The polycarbonate resin composition according to Item 6 or 7, wherein a difference between a refractive index of (C) the polyorganosiloxane and a refractive index of (A) the polycarbonate resin is 0.13 or less.
9. Amoldedbody, which is obtained by molding the polycarbonate resin composition of any one of Items 3 to 8.
10. The molded body according to Item 9, wherein the molded body comprises a light-guiding plate.
11. Abisphenol A, whichhas an isopropenylphenol concentration of 150 ppm by mass or less within 1 hour after granulation and under an ambient temperature condition of from 10°C to 50°C.
12. A method of producing a polycarbonate resin, comprising using, as a dihydric phenol serving as a raw material, a bisphenol A having an isopropenylphenol concentration of 150 ppm by mass or less within 1 hour after granulation and under an ambient temperature condition of from 10°C to 50°C.

ADVANTAGEOUS EFFECTS OF INVENTION

According to the present invention, there is provided a polycarbonate resin composition that can provide a molded body having a satisfactory hue (low YI value).

DESCRIPTION OF EMBODIMENTS

<Polycarbonate Resin>

The present invention relates to a polycarbonate resin, including, as a dihydric phenol serving as a raw material, a bisphenol A having an isopropenylphenol concentration of 150 ppm by mass or less within 1 hour after granulation and under an ambient temperature condition of from 10°C to 50°C.

The isopropenylphenol concentration can be measured by, for example, high performance liquid chromatography (hereinafter sometimes abbreviated as "HPLC"). When the isopropenylphenol concentration is more than 150 ppm by mass, the hue of a polycarbonate resin (A) using such a bisphenol A as a raw material deteriorates. The isopropenylphenol concentration of bisphenol A is preferably 100 ppm by mass or less, more preferably 50 ppm by mass or less. The phrase "after granulation" as used herein refers to the time point at which the bisphenol A in a molten state is brought into contact with cooling means, such as a cooling gas.

In the present invention, the isopropenylphenol concentration of the bisphenol A is a value measured for the bisphenol A within 1 hour after the granulation and under an ambient temperature condition of from 10°C to 50°C, preferably from 15°C to 40°C, more preferably from 20°C to 30°C. Time and temperature for the measurement of the isopropenylphenol concentration need to be set to fall within specific ranges because the isopropenylphenol concentration becomes lower with time and temperature. More specifically, the isopropenylphenol concentration in the present invention is a value measured for the bisphenol A 1 hour after the granulation and at an ambient temperature of 30°C.

An example of a method of producing the bisphenol A to be used as a raw material for the polycarbonate resin of the present invention is given below, but the present invention is not limited to the method.

The bisphenol A can be obtained by a product ion method including, for example, (1) a condensation reaction step of subj ecting an excess amount of phenol and acetone to a condensation reaction in the presence of an acid catalyst, (2) a concentration step of concentrating the reaction mixture obtained in the step (1), (3) a crystallization/solid-liquid separation step of cooling the concentrated reaction mixture obtained in the step (2) to crystallize an adduct of the bisphenol A and phenol to separate the mixture into the adduct and a mother liquid, (4) an adduct decomposition step of removing phenol from the adduct of the bisphenol A and phenol obtained in the step (3) to provide a bisphenol A molten liquid, and (5) a granulation step of granulating the bisphenol A molten liquid obtained in the step (4) to provide a granulated product.

A method of controlling the isopropenylphenol concentration can be, for example, as follows: a free acid removal step is newly arranged at an arbitrary place in the production process for the bisphenol A in accordance with the isopropenylphenol concentration to reduce the concentration of a free acid in the adduct crystal of the bisphenol A and phenol (hereinafter sometimes referred to as "adduct crystal") ; or the amount of a washing liquid in (3) the crystallization/solid-liquid separation step is changed to remove the free acid adhering to the adduct crystal of the bisphenol A and phenol. The isopropenylphenol concentration of the bisphenol A obtained by decomposing the adduct crystal can be controlled to 150 ppm by mass or less by sufficiently reducing the concentration of the free acid incorporated into and/or adhering to the adduct crystal. In addition, the above-mentioned method can not only control the isopropenylphenol concentration but also reduce the amount of other impurities, such as the 2,4-isomer of the bisphenol A.

When the isopropenylphenol concentration increases, the quality of the bisphenol A can be satisfactorily maintained by changing a condition for the production process so that the isopropenylphenol concentration may reduce (e.g., arranging the free acid removal step and/or increasing the amount of the washing liquid in the crystallization/solid-liquid separation step). In addition, when the isopropenylphenol concentration reduces, an unnecessary production process or the like can be stopped.

Isopropenylphenol is represented by the following general formula (1).

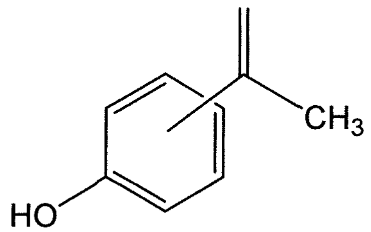

(1)

The viscosity-average molecular weight of the polycarbonate resin of the present invention is preferably from 9,000 to 17,500, more preferably from 11,000 to 15,500. The case where the viscosity-average molecular weight of the polycarbonate resin falls within the range is preferred because of the following reasons: a molding temperature suitable for the size of a molded article can be set, and hence the molded article does not have any yellow tint; and a molded

body retaining a strength can be obtained.

The viscosity-average molecular weight is a value calculated from the equation $[\eta] = 1.23\times10^{-5}Mv^{0.83}$ by using a limiting viscosity [η] determined through the measurement of the viscosity of a methylene chloride solution at 20°C with an Ubbelohde-type viscometer.

The content of methylene chloride in the polycarbonate resin of the present invention is preferably 200 ppm by mass or less, more preferably 50 ppm by mass or less, still more preferably 20 ppm by mass or less. When the polycarbonate resin is produced by an interfacial condensation reaction method, methylene chloride having a low boiling point is used as an organic solvent. However, it has been known that unless the methylene chloride is sufficiently removed in a posttreatment, the methylene chloride remains as an impurity in the polycarbonate resin and hence the yellowing of the resin progresses. In addition, the corrosion of a die due to the chloride is liable to occur and the surface of the die is roughened by the corrosion. Accordingly, continuous use of the die may reduce the light-guiding property of the product.

The polycarbonate resin of the present invention can be produced by a reaction between: the bisphenol A having an isopropenylphenol concentration of 150 ppm by mass or less within 1 hour after the granulation and under an ambient temperature condition of from 10°C to 50°C; and a carbonate precursor. The reaction is not particularly limited and a known method can be adopted therefor. However, the reaction is preferably performed by an interfacial polymerization method in the presence of an aqueous solution of an alkali compound and a water-insoluble organic solvent. The reaction can be performed in the presence of a polymerization catalyst as required.

Examples of the alkaline compound include: alkali metal hydroxides, such as sodium hydroxide and potassium hydroxide; and alkaline earth metal hydroxides, such as magnesium hydroxide and calcium hydroxide. Among them, an alkali metal hydroxide is preferred, and sodium hydroxide is more preferred. The dihydric phenol-based compound is preferably used as a mixture with the alkaline compound aqueous solution.

As the water-insoluble organic solvent, for example, a halogenated hydrocarbon, such as methylene chloride, chlorobenzene, or chloroform, is preferred, and methylene chloride is more preferred.

Examples of the polymerization catalyst include tertiary amines and quaternary ammonium salts. Examples of the tertiary amine include trimethylamine, triethylamine, and tripropylamine. Examples of the quaternary ammonium salt include trimethylbenzylammonium chloride and triethylammonium chloride. As the polymerization catalyst, a tertiary amine is preferred, and triethylamine is more preferred.

In addition, a molecular weight modifier may be used, as necessary. The molecular weight modifier is not particularly limited as long as the modifier is a monohydric phenol, and examples thereof include phenol, *o*-*n*-butylphenol, *m*-*n*-butylphenol, *p*-*n*-butylphenol, *o*-isobutylphenol, *m*-isobutylphenol, *p*-isobutylphenol, *o*-*t*-butylphenol, *m*-*t*-butylphenol, *p*-*t*-butylphenol, *o*-n-pentylphenol, *m*-*n*-pentylphenol, *p*-*n*-pentylphenol, *o*-*n*-hexylphenol, *m*-*n*-hexylphenol, *p*-*n*-hexylphenol, *p*-*t*-octylphenol, o-cyclohexylphenol, *m*-cyclohexylphenol, *p*-cyclohexylphenol, o-phenylphenol, *m*-phenylphenol, *p*-phenylphenol, *o*-*n*-nonylphenol, *m*-*n*-nonylphenol, *p*-*n*-nonylphenol, *o*-cumylphenol, *m*-cumylphenol, *p*-cumylphenol, o-naphthylphenol, m-naphthylphenol, *p*-naphthylphenol, 2,5-di-*t*-butylphenol, 2,4-di-*t*-butylphenol, 3,5-di-*t*-butylphenol, 2,5-dicumylphenol, 3,5-dicumylphenol, p-cresol, a monoalkylphenol having a linear or branched alkyl group having 12 to 35 carbon atoms on average at the ortho-, meta-, or para-position, 3-pentadecylphenol, 9-(4-hydroxyphenyl)-9-(4-methoxyphenyl)fluorene, 9-(4-hydroxy-3-methylphenyl)-9-(4-methoxy-3-methylphenyl)fluor ene, and 4-(1-adamantyl)phenol. Among them, *p*-t-butylphenol, *p*-cumylphenol, and *p*-phenylphenol are preferred, and *p*-*t*-butylphenol is more preferred.

For example, the following phase-transfer catalyst may be preferably used as the catalyst: a tertiary amine or a salt thereof, a quaternary ammonium salt, or a quaternary phosphonium salt. Examples of the tertiary amine include triethylamine, tributylamine, N,N-dimethylcyclohexylamine, pyridine, and dimethylaniline. In addition, examples of the tertiary amine salt include hydrochlorides and bromates of those tertiary amines. Examples of the quaternary ammonium salt include trimethylbenzylammonium chloride, triethylbenzylammonium chloride, tributylbenzylammonium chloride, trioctylmethylammonium chloride, tetrabutylammonium chloride, and tetrabutylammonium bromide. Examples of the quaternary phosphonium salt include tetrabutylphosphonium chloride and tetrabutylphosphonium bromide. Each of those catalysts may be used alone, or two or more kinds thereof may be used in combination. Among the catalysts, tertiary amines are preferred, and triethylamine is particularly suitable.

## <Polycarbonate Resin Composition>

### [(A) Polycarbonate Resin]

In another aspect of the present invention, there is provided a polycarbonate resin composition obtained by blending (A) a polycarbonate resin containing the polycarbonate resin of the present invention with (B) a phosphorus-based antioxidant to be described later.

The polycarbonate resin composition of the present invention may contain, as (A) the polycarbonate resin, a polycarbonate resin except the polycarbonate resin of the present invention to the extent that the resin does not affect

the hue, transparency, mechanical characteristics, and the like of the composition. In that case, the ratio of the polycarbonate resin of the present invention in (A) the polycarbonate resin is preferably 60 mass% or more, more preferably 80 mass% or more, still more preferably 100 mass%. When two or more kinds of polycarbonate resins are used as a mixture, the polycarbonate resins are desirably used after the viscosity-average molecular weight of the entirety of the polycarbonate resins has been adjusted to fall within the above-mentioned range.

[(B) Phosphorus-based Antioxidant]

The polycarbonate resin composition of the present invention is obtained by blending 100 parts by mass of the polycarbonate resin with 100 ppm by mass to 1,500 ppm by mass, preferably 300 ppm by mass to 1,200 ppm by mass of (B) the phosphorus-based antioxidant. When the amount of the antioxidant is less than 100 ppm by mass, an effect as an antioxidant is not sufficient and hence an increase in YI value cannot be suppressed. Meanwhile, when the amount is more than 1, 500 ppm by mass, the decomposition of the polycarbonate resin may progress owing to an acid generated by the decomposition of the antioxidant.

For example, the following antioxidant may be used as the phosphorus-based antioxidant: phosphorous acid, phosphonous acid, phosphonic acid, or esters thereof, or a tertiary phosphine. Among them, a phosphorous acid ester having a pentaerythritol structure represented by the following general formula (2) is preferred.

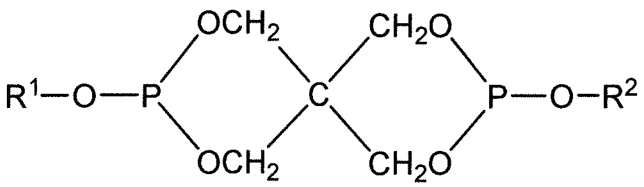

(2)

In the general formula (2), $R^1$ and $R^2$ each represent hydrogen, an alkyl group, a cycloalkyl group, or an aryl group. The cycloalkyl group and the aryl group may each be substituted by an alkyl group. When $R^1$ and $R^2$ each represent an aryl group, $R^1$ and $R^2$ each preferably represent an aryl group represented by the following general formula (a), (b), or (c).

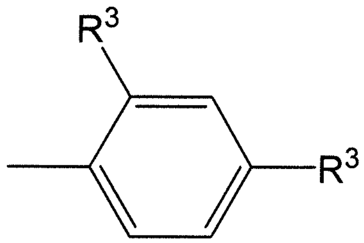

(a)

[In the formula (a), $R^3$ represents an alkyl group having 1 to 10 carbon atoms.]

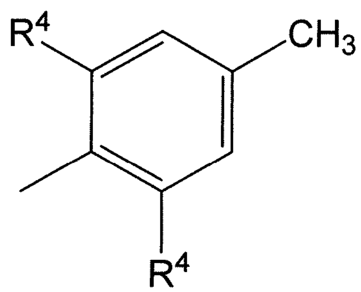

(b)

[In the formula (b), $R^4$ represents an alkyl group having 1 to 10 carbon atoms.]

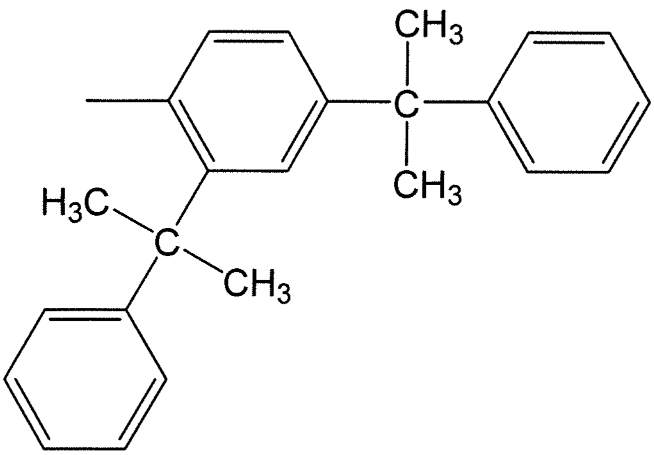

(C)

Specific examples thereof may include bis(2,6-di-*tert*-butyl-4-methylphenyl)pentaerythritol diphosphite represented by the following formula (3), bis(2,4-dicumylphenyl)pentaerythritol diphosphite represented by the following formula (4), and compounds represented by the following formulae (5) to (8).

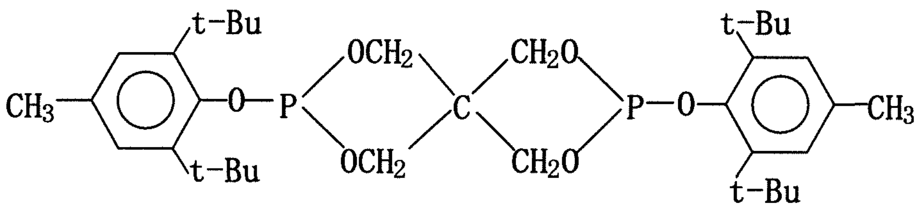

(3)

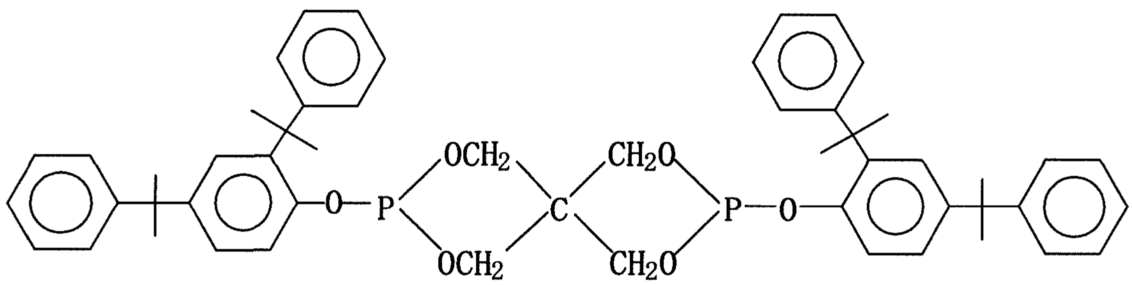

(4)

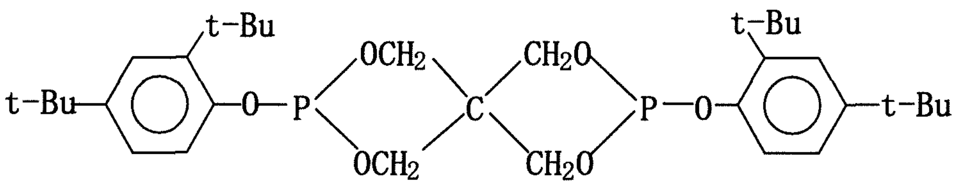

(5)

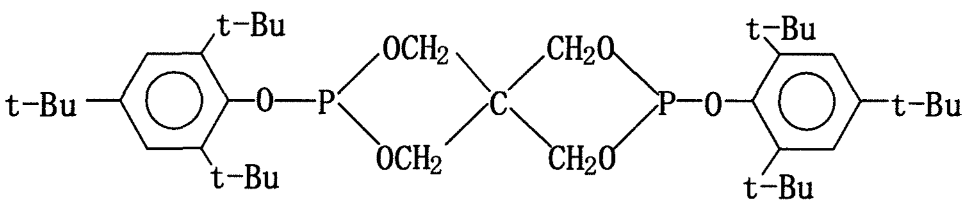

(6)

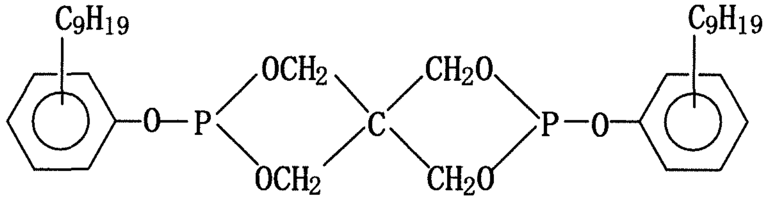

(7)

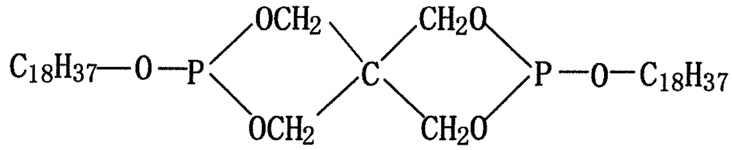

(8)

Bis(2,6-di-*tert*-butyl-4-methylphenyl)pentaerythritol diphosphite represented by the formula (3) [such as ADK STAB PEP-36 : manufactured by ADEKA Corporation] and/or bis(2,4-dicumylphenyl)pentaerythritol diphosphite represented by the formula (4) [such as Doverphos S-9228PC: manufactured by Dover Chemical Corporation] is suitable in the present invention.

[(C) Polyorganosiloxane having Functional Group]

In addition, preferably 200 ppm by mass to 1,500 ppm by mass, more preferably 300 ppm by mass to 1,200 ppm by mass of (C) a polyorganosiloxane having a functional group can be blended into the polycarbonate resin composition of the present invention with respect to 100 parts by mass of (A) the polycarbonate resin. Blending (C) the polyorganosiloxane having a functional group at a content in the range of from 200 ppm by mass to 1, 500 ppm by mass can improve the releasability of the composition together with any other component. Further, the blending can significantly reduce the occurrence of a silver streak and the amount of a die deposit even under a high-temperature molding condition largely exceeding 300°C, especially under a continuous molding condition.

The polyorganosiloxane having a functional group preferably has, as the functional group, at least one of functional group selected from the group consisting of an alkoxy group, an aryloxy group, a polyoxyalkylene group, a carboxyl group, a silanol group, an amino group, a mercapto group, an epoxy group, and a vinyl group.

The viscosity of the polyorganosiloxane having a functional group at 25°C is preferably 10 $mm^2$/sec or more from the viewpoint of its lubricating effect, and is more preferably 200 $mm^2$/sec or less from the viewpoint of its dispersibility in the polycarbonate resin. From the foregoing viewpoints, the viscosity range of the polyorganosiloxane having a functional group is still more preferably from 20 $mm^2$/sec to 150 $mm^2$/sec, particularly preferably from 40 $mm^2$/sec to 120 $mm^2$/sec.

In order to prevent the transparency of the polycarbonate resin composition from reducing, a difference between the refractive index of the polyorganosiloxane having a functional group and the refractive index of the polycarbonate resin is preferably reduced to the extent possible, for example, to 0.13 or less. The refractive index of the polyorganosiloxane having a functional group is preferably 1.45 or more, more preferably 1.50 or more, still more preferably 1.52 or more because the refractive index of the polycarbonate resin is about 1.58.

In the polycarbonate resincompositionof the present invention, (D) an acrylic resin can be added as required. The acrylic resin refers to a polymer having, as a repeating unit, at least one kind selected from monomer units, i.e., acrylic acid, an acrylate, acrylonitrile, and derivatives thereof, and refers to a homopolymer or a copolymer with styrene, butadiene, or the like. Specific examples thereof include polyacrylic acid, polymethyl methacrylate (PMMA), polyacrylonitrile, an ethyl acrylate-2-chloroethyl acrylate copolymer, a *n*-butyl acrylate-acrylonitrile copolymer, an acrylonitrile-styrene copolymer, an acrylonitrile-butadiene copolymer, and an acrylonitrile-butadiene-styrene copolymer. Among them, polymethyl methacrylate (PMMA) may be particularly suitably used.

The polymethyl methacrylate (PMMA) may be a known product, and is typically produced by subjecting a methyl methacrylate monomer to bulk polymerization in the presence of a peroxide and an azo-based polymerization initiator. Its molecular weight is preferably from about 1,000 to about 200, 000 from the viewpoint of its compatibility with (A) the polycarbonate resin.

The blending amount of the acrylic thermoplastic resin is typically from 0.001 part by mass to 1 part by mass, preferably from 0.005 part by mass to 0.5 part by mass, more preferably from 0.01 part by mass to 0.1 part by mass with respect to 100 parts by mass of (A) the polycarbonate resin. When the blending amount of the acrylic thermoplastic resin is 0.001 part by mass or more, a polycarbonate-based resin composition having a satisfactory light-guiding property is obtained. In addition, when the blending amount is 1 part by mass or less, a polycarbonate-based resin composition having a satisfactory light-guiding property can be obtained because no phase separation of the acrylic resin component

occurs and hence the composition does not become opaque.

[Additive]

Any other resin or an additive, such as an oxidation inhibitor, a weathering agent, a lubricant, a release agent, a plasticizer, a flowability improver, or a charge inhibitor, can be added to the polycarbonate resin composition of the present invention at the time of its mixing or at the time of its molding to the extent that its physical properties are not impaired.

[Method of producing Polycarbonate Resin Composition]

A method of producing the polycarbonate resin composition of the present invention is, for example, a method involving melting and kneading the respective components by a conventionally known method.

For example, the following method is appropriately selected: the respective components are dispersed and mixed with a high-speed mixer typif iedby a Turnbull mixer, a Henschel mixer, a ribbonblender, or a super mixer, and then the resultant is melted and kneaded with an extruder, a Banbury mixer, a roll, or the like.

The polycarbonate resin composition of the present invention can be suitably used in molding at high temperature exceeding 300°C for producing a thin-walled molded article.

A molding method involving using the polycarbonate resin composition of the present invention is not particularly limited, and a molding method such as injection molding, injection compression molding, extrusion molding, or blow molding can be applied.

A molded article obtained by molding the polycarbonate resin composition of the present invention has a low YI value and is excellent in hue. Accordingly, the molded article can also be suitably used in a light-guiding member, such as a light-guiding plate having a long light-guiding length in which a required light transmission length is not the thickness direction of a thin-walled molded body but the longitudinal direction of a planar molded body. The molded body obtained by molding the polycarbonate resin composition of the present invention is preferably a light-guiding member, more preferably a light-guiding plate.

EXAMPLES

The present invention is described more specifically below by way of Examples. However, the present invention is by no means limited by these examples. Respective components, and measurement methods (calculation methods) for physical property values and the like used in Examples are as described below.

[(B) Phosphorus-based Antioxidant]

(B-1): ADK STAB PEP-36

[bis(2,6-di-*tert*-butyl-4-methylphenyl)pentaerythritol diphosphite manufactured by ADEKA Corporation]

(B-2): Doverphos S-9228PC [bis(2,4-dicumylphenyl)pentaerythritol diphosphite manufactured by Dover Chemical Corporation]

[(C) Polyorganosiloxane having Functional Group]

KR-511 [manufactured by Shin-Etsu Chemical Co., Ltd.,
organopolysiloxane having a methoxy group], refractive index: 1.52,
viscosity: 25°C, 85.8 $mm^2$/sec

[(D) Acrylic Thermoplastic Resin]

DIANALBR-83 [manufactured by Mitsubishi Rayon Co. , Ltd. , polymethyl methacrylate (PMMA)]

(1) Measurement of YI Value

Evaluation of Molded Body YI

A YI value was measured with a spectrophotometer "U-4100" (manufactured by Hitachi High-Technologies Corporation) under the conditions of a C light source and a two-degree field of view. The YI value of a molded body obtained by molding a polycarbonate resin composition was corrected to a YI value at a thickness of 60 mm by using the thickness correction function of a software included in the apparatus ("UV Solutions Program No. 1344311-16").

Acceptance criteria for the YI value of the molded body obtained by molding the polycarbonate resin composition were set as follows: 18.0 or less after the correction to a YI value at a thickness of 60 mm for a molded body molded at 280°C and 19.0 or less after the correction to a YI value at a thickness of 60 mm for a molded body molded at 360°C.

(2) Method of measuring Viscosity-average Molecular Weight

The limiting viscosity [η] of a methylene chloride solution at 20°C was measured with an Ubbelohde-type viscosity tube, and a viscosity-average molecular weight was calculated from the following relational expression (Schnell's equation).

$$[\eta] = 1.23\times10^{-5}\times Mv^{0.83}$$

Example 1

A bisphenol A obtained after granulation was stored under a room temperature condition (30°C), and the bisphenol A after a lapse of 1 hour from the granulation was analyzed by high performance liquid chromatography. In the present invention, the time point at which a bisphenol A molten liquid flows out of a granulation nozzle is defined as 0 hours after the granulation. A high performance liquid chromatograph (manufactured by Waters, model: 2695, column: manufactured by GL Sciences Inc. , Inertsil (trademark) ODS-3V) was used in the analysis. The mobile phase which was 25 mass% aqueous solution of acetonitrile kept for 45 minutes, and then analysis mode changed to 3.5 mass%/min gradient mode. After having reached 100 mass% acetonitrile, this condition was retained for 5 minutes. The injection amount of the sample was set to 5.0 μL, a column temperature was set to 40°C, a flow rate was set to 1.0 mL/min, and an analysis wavelength was set to 277 nm.

A polycarbonate resin was produced by using the bisphenol A that was found to have an IPP concentration 1 hour after the granulation of 27 ppm by mass as a result of the HPLC measurement as a dihydric phenol serving as a raw material in accordance with the following production example.

Production Example: Production of Bisphenol A Polycarbonate Resin

(1) Polycarbonate Oligomer Synthesis Step

To 5.6 mass% aqueous sodium hydroxide, sodium dithionite was added in an amount of 2,000 ppm by mass relative to bisphenol A (hereinafter sometimes abbreviated as "BPA") to be dissolved later, and BPA obtained in this example was then dissolved therein so that the concentration of BPA became 13.5 mass%, to thereby prepare a solution of BPA in aqueous sodium hydroxide. The solution of BPA in aqueous sodium hydroxide, methylene chloride, and phosgene were continuously passed through a tubular reactor having an inner diameter of 6 mm and a tube length of 30 m at flow rates of 40 L/hr, 15 L/hr, and 4.0 kg/hr, respectively. The tubular reactor had a jacket portion, and cooling water was passed through the jacket to keep the reaction liquid at a temperature of 40°C or less.

The reaction liquid that had exited the tubular reactor was continuously introduced into a baffled tank-type reactor having an internal volume of 40 L and provided with a sweptback blade, and then, 2.8 L/hr of the solution of BPA in aqueous sodium hydroxide, 0.07 L/hr of 25 mass% aqueous sodium hydroxide, 17 L/hr of water, and 0.64 L/hr of a 1 mass% triethylamine aqueous solution were further added to the reactor to perform a reaction.

The reaction liquid overflown from the tank-type reactor was continuously taken out and left to stand still to separate and remove an aqueous phase, and a methylene chloride phase was then collected. The concentration of the obtained polycarbonate oligomer was 325 g/L, and the concentration of a chloroformate group thereof was 0.77 mol/L.

(2) Polycarbonate Polymerization Step

After the temperature of the cooling solvent of a 50-liter tank-type reactor provided with a baffle board, a paddle-type stirring blade, and a cooling jacket had become 20°C or less, 15 L of the oligomer solution, 8.9 L of methylene chloride, 192 g of *p-tert*-butylphenol, 0.7 mL of triethylamine, and a solution of BPA in aqueous sodium hydroxide (obtained by dissolving 1,185 g of BPA in an aqueous solution obtained by dissolving 647 g of NaOH and 2,000 ppm by mass of sodium dithionite with respect to BPA to be dissolved later in 9.5 L of water) were added to perform a polymerization reaction for 30 minutes. After that, 0.8 mL of triethylamine was added to the resultant and the mixture was further stirred for 30 minutes.

15 Liters of methylene chloride was added to the mixture for dilution, and then the diluted mixture was separated into an organic phase containing a polycarbonate resin, and an aqueous phase containing excess BPA and NaOH, followed by the isolation of the organic phase. The resultant solution of the polycarbonate resin in methylene chloride was sequentially washed with 15 vol% each of 0.03 mol/L aqueous NaOH and 0.2 mol/L hydrochloric acid with respect to the solution. Next, washing with pure water was repeated until an electric conductivity in the aqueous phase after the washing became 0.05 μS/m or less. A solution of the polycarbonate resin in dichloromethane obtained by the washing was concentrated and pulverized, and the resultant flake was dried under reduced pressure at 100°C to provide (A) a polycarbonate resin flake. The flake had a viscosity-average molecular weight of 15,300. The refractive index of the polycarbonate resin was 1.58.

<Evaluation>

[Preparation of Polycarbonate Resin Composition/YI Value of Molded Body to be obtained]

100 Parts by mass of (A) the polycarbonate resin flake thus obtained was dry-blended with 500 ppm by mass of (B-1) the phosphorus-based antioxidant, and then the blend was melted and kneaded with a single screw extruder at a cylinder temperature of 260°C. The resultant strand was passed through a water tank to be cooled, and was then pelletized. The resin pellet was dried at 110°C for 5 hours, and then a flat plate-shaped molded body measuring 25 mm by 35 mm by 3.0 mm thick was produced from the pellet with an injection molding machine "Toshiba EC40N" (manufactured by Toshiba Machine Co., Ltd., clamping force: 40 tons) at a cylinder temperature setting of 360°C and a die temperature of 80°C for a cycle time of 25 seconds, followed by the measurement of its YI value. The result is shown in Table 1.

Example 2

A polycarbonate resin flake was produced in accordance with Production Example in the same manner as in Example 1 except that a bisphenol A having an IPP concentration 1 hour after granulation of 31 ppm by mass was used, and then its viscosity-average molecular weight was measured. The refractive index of the polycarbonate resin was 1.58. In addition, in the same manner as in Example 1, a polycarbonate resin composition was prepared and then a flat plate-shaped molded body was produced, followed by the measurement of its YI value. The result is shown in Table 1.

Example 3

A polycarbonate resin flake was produced in accordance with Production Example in the same manner as in Example 1 except that a bisphenol A having an IPP concentration 1 hour after granulation of 43 ppm by mass was used, and then its viscosity-average molecular weight was measured. The refractive index of the polycarbonate resin was 1.58. In addition, a polycarbonate resin composition was prepared in the same manner as in Example 1 except that 100 parts by mass of (A) the polycarbonate resin flake was dry-blended with 500 ppm by mass of (C) the polyorganosiloxane having a functional group together with the components (A) and (B-1). Aflatplate-shaped molded body was produced in the same manner as in Example 1 except that the cylinder temperature setting was changed to 280°C, and its YI value was measured. The result is shown in Table 2.

Example 4

A polycarbonate resin flake was produced in accordance with Production Example in the same manner as in Example 1 except that a bisphenol A having an IPP concentration 1 hour after granulation of 29 ppm by mass was used, and then its viscosity-average molecular weight was measured. The refractive index of the polycarbonate resin was 1.58. In addition, a polycarbonate resin composition was prepared in the same manner as in Example 1 except that: (B-2) the phosphorus-based antioxidant was used as an antioxidant; and 100 parts by mass of (A) the polycarbonate resin

flake was dry-blended with 500 ppm by mass of (C) the polyorganosiloxane having a functional group together with the components (A) and (B-2). Aflatplate-shaped molded body was produced in the same manner as in Example 1 at a cylinder temperature setting of 280°C or 360°C, and its YI value was measured. The results are shown in Tables 1 and 2.

Example 5

A polycarbonate resin flake was produced in accordance with Production Example in the same manner as in Example 1 except that a bisphenol A having an IPP concentration 1 hour after granulation of 29 ppm by mass was used, and then its viscosity-average molecular weight was measured. The refractive index of the polycarbonate resin was 1.58. In addition, a polycarbonate resin composition was prepared in the same manner as in Example 1 except that: (B-2) the phosphorus-based antioxidant was used as an antioxidant; and 100 parts by mass of (A) the polycarbonate resin flake was dry-blended with 500 ppm by mass of (C) the polyorganosiloxane having a functional group and 200 ppm by mass of (D) the acrylic nitrile resin together with the components (A) and (B-2). A flat plate-shaped molded body was produced in the same manner as in Example 1 at a cylinder temperature setting of 280°C or 360°C, and its YI value was measured. The results are shown in Tables 1 and 2.

Example 6

A polycarbonate resin flake was produced in accordance with Production Example in the same manner as in Example 1 except that a bisphenol A having an IPP concentration 1 hour after granulation of 120 ppm by mass was used, and then its viscosity-average molecular weight was measured. The refractive indexof the polycarbonate resin was 1.58. In addition, a polycarbonate resin composition was prepared in the same manner as in Example 1 except that 100 parts by mass of (A) the polycarbonate resin flake was dry-blended with 500 ppm by mass of (C) the polyorganosiloxane having a functional group together with the components (A) and (B-1). Aflatplate-shaped molded body was produced in the same manner as in Example 1 except that the cylinder temperature setting was changed to 280°C, and its YI value was measured. The result is shown in Table 2.

Comparative Example 1

A polycarbonate resin flake was produced in accordance with Production Example in the same manner as in Example 1 except that a bisphenol A having an IPP concentration 1 hour after granulation of 245 ppm by mass was used, and then its viscosity-average molecular weight was measured. The refractive index of the polycarbonate resin was 1.58. In addition, a polycarbonate resin composition was prepared in the same manner as in Example 1. A flat plate-shaped molded body was produced in the same manner as in Example 1 at a cylinder temperature setting of 280°C or 360°C, and its YI value was measured. The results are shown in Tables 1 and 2.

Table 1: Molding at 360°C

| | | Example 1 | Example 2 | Example 4 | Example 5 | Comparative Example 1 |
|---|---|---|---|---|---|---|
| (A) PC resin | Part(s) by mass | 100 | 100 | 100 | 100 | 100 |
| (B-1) Phosphorus-based antioxidant | ppm by mass | 500 | 500 | 0 | 0 | 500 |
| (B-2) Phosphorus-based antioxidant | ppm by mass | 0 | 0 | 500 | 500 | 0 |
| (C) Polyorganosiloxane | ppm by mass | - | - | 500 | 500 | - |
| (D) Acrylic resin | ppm by mass | - | - | - | 200 | - |
| IPP concentration | ppm by mass | 27 | 31 | 29 | 29 | 245 |
| Viscosity-average molecular weight | (Mv) | 15,300 | 15,300 | 14,800 | 14,800 | 15,100 |

(continued)

| | | Example 1 | Example 2 | Example 4 | Example 5 | Comparative Example 1 |
|---|---|---|---|---|---|---|
| Molded body YI (measuring 25 mm by 35 mm by 3.0 mm thick) (value converted in terms of a thickness of 60 mm) | 25 seconds @ 360°C | 17.3 | 17.4 | 16.8 | 15.7 | 19.8 |

Table 2: Molding at 280°C

| | | Example 3 | Example 4 | Example 5 | Example 6 | Comparative Example 1 |
|---|---|---|---|---|---|---|
| (A) PC resin | Part(s) by mass | 100 | 100 | 100 | 100 | 100 |
| (B-1) Phosphorus-based antioxidant | ppm by mass | 500 | 0 | 0 | 500 | 500 |
| (B-2) Phosphorus-based antioxidant | ppm by mass | 0 | 500 | 500 | 0 | 0 |
| (C) Polyorganosiloxane | ppm by mass | 500 | 500 | 500 | 500 | - |
| (D) Acrylic resin | ppm by mass | - | - | 200 | - | - |
| IPP concentration | ppm by mass | 43 | 29 | 29 | 120 | 245 |
| Viscosity-average molecular weight | (Mv) | 15,300 | 14,800 | 14,800 | 15,200 | 15,100 |
| Molded body YI (measuring 25 mm by 35 mm by 3.0 mm thick) (value converted in terms of a thickness of 60 mm) | 25 seconds @ 280°C | 15.0 | 15.4 | 14.4 | 16.0 | 18.8 |

It is understood from Examples 1 and 2, and 4 and 5 of Table 1 that the polycarbonate resin composition of the present invention in which the dihydric phenol serving as a raw material is the bisphenol A having an isopropenylphenol concentration of 150 ppm by mass or less within 1 hour after the granulation and after the storage at 30°C is excellent in hue because the YI value after the correction to a YI value at a thickness of 60 mm is 19.0 or less even in the high-temperature molding at 360°C. It is understood from Examples 3 to 6 of Table 2 that even when the polycarbonate resin composition of the present invention is molded at 280°C, the YI value after the correction to a YI value at a thickness of 60 mm is 18.0 or less, and hence the composition is excellent in hue. In contrast, in Comparative Example 1 in which the bisphenol A having an isopropenylphenol concentration within 1 hour after the granulation and after the storage at 30°C of more than 150 ppm by mass is used as the dihydric phenol serving as a raw material, the acceptance criteria are not satisfied in both the molding at 360°C and the molding at 280°C.

INDUSTRIAL APPLICABILITY

According to the present invention, there is provided a polycarbonate resin composition that can provide a molded body having a satisfactory hue (low YI value). The polycarbonate resin composition can be suitably used as a light-guiding member.

## Claims

**1.** A polycarbonate resin, comprising, as a dihydric phenol serving as a raw material, a bisphenol A having an isopropenylphenol concentration of 150 ppm by mass or less within 1 hour after granulation and under an ambient temperature condition of from 10°C to 50°C.

**2.** The polycarbonate resin according to claim 1, wherein the polycarbonate resin has a viscosity-average molecular weight of from 9,000 to 17,500.

**3.** A polycarbonate resin composition, comprising 100 parts by mass of (A) a polycarbonate resin containing 60 mass% or more of the polycarbonate resin of claim 1 or 2 and 100 ppm by mass to 1,500 ppm by mass of (B) a phosphorus-based antioxidant.

**4.** The polycarbonate resin composition according to claim 3, wherein (B) the phosphorus-based antioxidant has a pentaerythritol structure.

**5.** The polycarbonate resin composition according to claim 3 or 4, wherein (B) the phosphorus-based antioxidant comprises bis(2,6-di-*tert*-butyl-4-methylphenyl)pentaerythritol diphosphite and/or bis(2,4-dicumylphenyl)pentaerythritol diphosphite.

**6.** The polycarbonate resin composition according to any one of claims 3 to 5, further comprising, with respect to 100 parts by mass of (A) the polycarbonate resin, 200 ppm by mass to 1,500 ppm by mass of (C) a polyorganosiloxane having a functional group.

**7.** The polycarbonate resin composition according to claim 6, wherein the functional group comprises at least one kind selected from the group consisting of an alkoxy group, an aryloxy group, a polyoxyalkylene group, a carboxyl group, a silanol group, an amino group, a mercapto group, an epoxy group, and a vinyl group.

**8.** The polycarbonate resin composition according to claim 6 or 7, wherein a difference between a refractive index of (C) the polyorganosiloxane and a refractive index of (A) the polycarbonate resin is 0.13 or less.

**9.** A molded body, which is obtained by molding the polycarbonate resin composition of any one of claims 3 to 8.

**10.** The molded body according to claim 9, wherein the molded body comprises a light-guiding plate.

**11.** A bisphenol A, which has an isopropenylphenol concentration of 150 ppm by mass or less within 1 hour after granulation and under an ambient temperature condition of from 10°C to 50°C.

**12.** A method of producing a polycarbonate resin, comprising using, as a dihydric phenol serving as a raw material, a bisphenol A having an isopropenylphenol concentration of 150 ppm by mass or less within 1 hour after granulation and under an ambient temperature condition of from 10°C to 50°C.

**INTERNATIONAL SEARCH REPORT**

International application No. PCT/JP2015/055090

A. CLASSIFICATION OF SUBJECT MATTER

*C08G64/04*(2006.01)i, *C07C39/16*(2006.01)i, *C08K5/524*(2006.01)i, *C08L69/00*(2006.01)i, *C08L83/04*(2006.01)i

According to International Patent Classification (IPC) or to both national classification and IPC

B. FIELDS SEARCHED

Minimum documentation searched (classification system followed by classification symbols)
C08G64/04, C07C39/16, C08K5/524, C08L69/00, C08L83/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2002-534402 A (Bayer AG.),<br>15 October 2002 (15.10.2002),<br>claims; examples<br>& US 6689464 B1 & EP 1140754 A1<br>& WO 2000/040533 A1 & DE 19900221 A<br>& AU 2435200 A & BR 7410 A<br>& CZ 20012413 A & TW 498062 B<br>& ID 30275 A & AT 316521 T<br>& ES 2255969 T & CN 1335830 A | 1,2<br>3-10,12 |
| X<br>Y | JP 2005-75736 A (Mitsubishi Chemical Corp.),<br>24 March 2005 (24.03.2005),<br>claims; paragraphs [0050], [0058], [0059], [0061]; examples<br>(Family: none) | 1-3,9,10,12<br>4-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 May 2015 (08.05.15) | 19 May 2015 (19.05.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

INTERNATIONAL SEARCH REPORT

International application No. PCT/JP2015/055090

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2010-275484 A (Idemitsu Kosan Co., Ltd.), 09 December 2010 (09.12.2010), claims; paragraph [0030]; examples & WO 2010/137611 A1 & TW 201105741 A & CN 102449067 A & KR 10-2012-0024664 A | 4-8,10 |
| A | JP 2006-335850 A (Mitsubishi Chemical Corp.), 14 December 2006 (14.12.2006), entire text (Family: none) | 1-10,12 |
| A | JP 2011-174031 A (Idemitsu Kosan Co., Ltd.), 08 September 2011 (08.09.2011), entire text & CN 102140237 A | 1-10,12 |
| A | JP 2013-227510 A (Mitsubishi Chemical Corp.), 07 November 2013 (07.11.2013), entire text; all drawings & WO 2013/147221 A1 & CN 104169326 A | 1-10,12 |

Form PCT/ISA/210 (continuation of second sheet) (July 2009)

**INTERNATIONAL SEARCH REPORT**

International application No.
PCT/JP2015/055090

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See extra sheet.

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
1-10 and 12

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT

International application No.
PCT/JP2015/055090

Continuation of Box No.III of continuation of first sheet(2)

(1) The inventions of claims 1-3, 9 and 10 lack novelty in the light of JP 2005-75736 A (Mitsubishi Chemical Corp.), 24 March 2005 (24.03.2005), and have no special technical feature.

Next, a special technical feature could be found in the invention of claim 4.

In addition, the technical feature common to the invention of claims 11 and 12 and the invention of claim 4 is "the dihydric phenol serving as a starting material, which is a bisphenol A having an isopropenyl phenol concentration of 150 ppm by mass or less within one hour after granulation at an ambient temperature of 10-50°C".

Consequently, the above-said technical feature cannot be considered to be a special technical feature, since the technical feature does not make a contribution over the prior art in the light of the contents disclosed in the above-said official document.

In addition, there is no other same or corresponding special technical feature between the inventions of claims 11, 12 and the invention of claim 4.

(2) The invention of claim 11 is not relevant to an invention which involves all of the matters to define the invention in claim 1 and which has a same category.

Further, as a result of the search which has been carried out with respect to inventions to be searched on the basis of special technical features, the invention of claim 11 is not relevant to an invention on which it is substantially possible to carry out a search without an additional prior-art search and judgment, and there is no other reason for that it can be considered that it is efficient to carry out a search thereon together with said inventions (JP 2002-534402 A (Bayer AG.), 15 October 2002 (15.10.2002) has been accidently found during the prior-art search on the invention of claim 1).

Further, the invention of claim 12 is relevant to an invention on which it is efficient to carry out a search together with the inventions to be searched on the basis of special technical features, and is therefore added to the subject to be searched.

Consequently, with respect to the invention of claim 11, the requirements other than the requirement of unity of invention have not been covered by this search.

(Invention 1) claims 1-10 and 12

A polycarbonate resin which uses a bisphenol A having an isopropenyl phenol concentration of 150 ppm by mass or less within one hour after granulation at an ambient temperature of 10-50°C as a dihydric phenol that serves as a starting material; and a method for producing the polycarbonate resin.

(Invention 2) claim 11

A bisphenol A having an isopropenyl phenol concentration of 150 ppm by mass or less within one hour after granulation at an ambient temperature of 10-50°C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4621312 B
- JP 2001344813 A
- JP 2002173530 A